# EUROPEAN PATENT APPLICATION

(11) **EP 4 497 813 A1**
(43) Date of publication of application: **29.01.2025**
(21) Application number: 23775025.2
(22) Date of filing: 23.03.2023
(51) Int. Cl.: C12M 1/04, C12M 1/34, C12M 1/36

(54) **CELL CULTURE APPARATUS**

(30) Priority: 23.03.2022 JP 2022046917
(71) Applicant: Fujimori Kogyo Co., Ltd., Tokyo 112-0002 (JP)
(72) Inventor: TOKURA, Tomohiro, Tokyo 112-0002 (JP); MATSUDA, Hiroyuki, Tokyo 112-0002 (JP); ITO, Syouta, Tokyo 112-0002 (JP)
(74) Representative: Lavoix
(86) International application number: PCT/JP2023/011517
(87) International publication number: WO 2023/182431

(57) **Abstract**

A cell culture device (10) includes a culture tank (1), a drive unit (2), and a controller (3). The culture tank (1) stores a culture solution (11) containing cells. The drive unit (2) causes the culture tank (1) to perform a shaking operation and thereby causes the culture solution (11) to flow while being into contact with a gas (12) in the culture tank (1). The controller (3) controls an operation of the drive unit (2) such that a shaking speed of the culture tank (1) increases continuously or intermittently along with the passage of time.

## Description

### TECHNICAL FIELD

The present invention relates to a cell culture device.

Priority is claimed on Japanese Patent Application No. 2022-46917 filed on March 23, 2022, the contents of which are incorporated herein by reference.

### BACKGROUND ART

A culture device for culturing animal cells and the like is widely used in production of various substances useful as pharmaceuticals, foods, cosmetics, and their raw materials. The culture device includes, for example, a liquid container and a shaking device that shakes the liquid container (see, for example, Patent Document 1). In the culture device, a culture solution in the liquid container is shaken by shaking the liquid container. Therefore, oxygen in the air is taken into the culture solution from a liquid surface.

### Citation List

### Patent Document

Patent Document 1: Japanese Unexamined Patent Application, First Publication No. 2021-145566

### SUMMARY OF INVENTION

### Technical Problem

In culturing animal cells or the like, a dissolved oxygen concentration in the culture solution has a great influence on a physiological state of cells. In the above-described culture device, the dissolved oxygen concentration of the culture solution tends to increase, and it is difficult to adjust the dissolved oxygen concentration appropriately. Particularly, in a tank shaking-type culture device, since a liquid surface of the culture solution moves significantly, an amount of oxygen supplied from the liquid surface of the culture solution tends to be higher than with other agitating methods, a dissolved oxygen concentration becomes too high during a period in which the number of cells is small at the beginning of the culture, and therefore there has been a problem in that cells are susceptible to oxygen damage.

An objective of one aspect of the invention is to provide a cell culture device capable of appropriately adjusting a dissolved oxygen concentration of a culture solution. Solution to Problem

One aspect of the invention provides a cell culture device including a culture tank that stores a culture solution containing cells, a drive unit that causes the culture tank to perform a shaking operation and thereby causes the culture solution to flow while being into contact with a gas in the culture tank, and a controller that controls an operation of the drive unit such that a shaking speed of the culture tank increases continuously or intermittently along with the passage of time.

It is preferable that the cell culture device further include a dissolved oxygen meter that measures a dissolved oxygen concentration of the culture solution, in which the controller controls the shaking speed of the culture tank based on a detected value of the dissolved oxygen concentration by the dissolved oxygen meter.

It is preferable that the cell culture device further include a plurality of supply systems that supply oxygen to the culture tank, in which the plurality of supply systems include a first supply system and a second supply system, and the controller drives the first supply system in accordance with the fact that the shaking speed of the culture tank has reached a specific value, and drives the second supply system in accordance with the fact that an amount of oxygen supplied by the first supply system has reached a maximum or set value.

It is preferable that the second supply system have a higher capacity to supply oxygen dissolved in the culture solution than that of the first supply system.

It is preferable that the cell culture device further include a plurality of supply systems that supply oxygen to the culture tank, and a dissolved oxygen meter that measures a dissolved oxygen concentration of the culture solution, in which the supply systems include a first supply system and a second supply system, and the controller controls driving and stopping of the first supply system based on a detected value of the dissolved oxygen concentration by the dissolved oxygen meter, and controls driving and stopping of the second supply system based on a detected value of the dissolved oxygen concentration by the dissolved oxygen meter.

It is preferable that the first supply system supply an oxygen-containing gas to a gas-phase space of the culture tank, and the second supply system include a gas disperser that supplies an oxygen-containing gas into the culture solution.

It is preferable that the cell culture device further include a third supply system that adjusts an oxygen concentration of the oxygen-containing gas supplied to the gas disperser.

Another aspect of the invention provides a cell culture method of causing the culture tank that stores the culture solution containing cells to perform a shaking operation and thereby causing the culture solution to flow while being into contact with a gas in the culture tank, and causing the culture tank to operate such that a shaking speed of the culture tank increases continuously or intermittently along with the passage of time when the culture tank performs the shaking operation.

### Advantageous Effects of Invention

According to one aspect of the invention, a dissolved oxygen concentration of the culture solution can be appropriately adjusted.

### BRIEF DESCRIPTION OF DRAWINGS

[FIG. 1] A schematic view showing a cell culture device of a first embodiment.
[FIG. 2] A diagram showing a first example of temporal change in shaking speed.
[FIG. 3] A diagram showing a second example of temporal change in shaking speed.
[FIG. 4] A diagram showing a third example of temporal change in shaking speed.
[FIG. 5] A schematic view showing a cell culture device of a second embodiment.
[FIG. 6] A schematic view showing a cell culture device of a third embodiment.
[FIG. 7] A schematic view showing a cell culture device of a fourth embodiment.

### DESCRIPTION OF EMBODIMENTS

Hereinafter, the invention will be described with reference to preferred embodiments with reference to the drawings.

### [Cell culture device] (First embodiment)

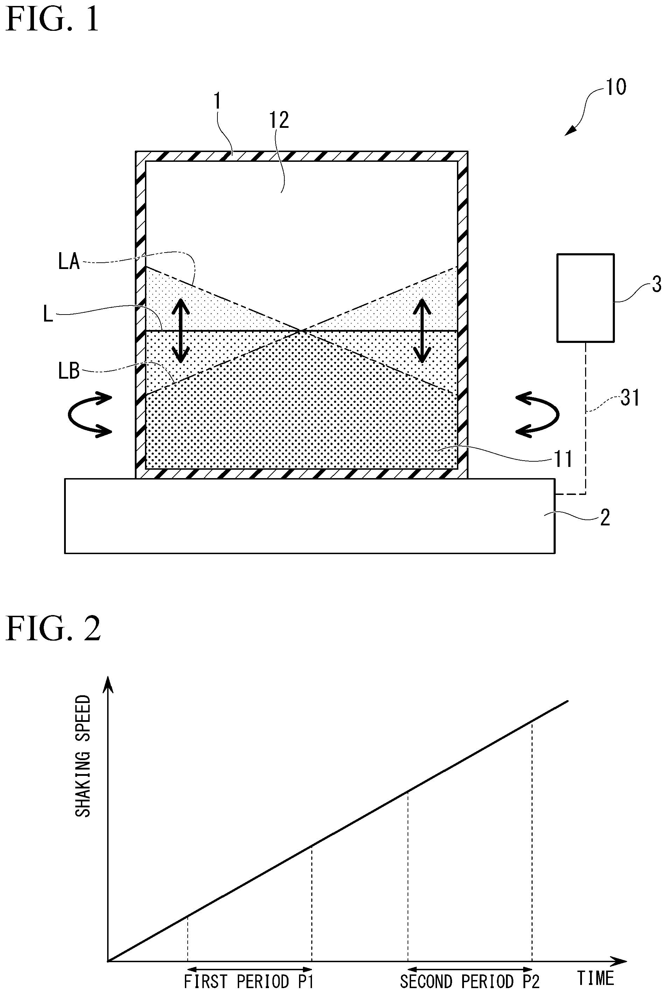

FIG. 1 is a schematic view showing a cell culture device 10 of a first embodiment.

As shown in FIG. 1, the cell culture device 10 includes a culture tank 1, a drive unit 2, and a controller 3.

The culture tank 1 can store a culture solution 11. The culture tank 1 may have a structure that opens to the outside air, but is preferably sealed. It is also possible to attach a single-use bag in the culture tank 1 to culture cells therein.

The culture solution 11 is, for example, a liquid culture medium containing cells. An amount of the culture solution 11 in the culture tank 1 is smaller than a total volume of an internal space of the culture tank 1. Therefore, a space (gas-phase space) is secured in the culture tank 1 above a liquid surface L of the culture solution 11. There is a gas 12 in the space. The gas 12 is an oxygen-containing gas such as air. Furthermore, an oxygen concentration of the gas 12 may be higher or lower than an oxygen concentration of air. An oxygen concentration of the oxygen-containing gas may be higher than 0% and 100% or lower. The oxygen concentration of the oxygen-containing gas may be, for example, approximately 21% to 100%. "%" of the oxygen concentration is a volume ratio (vol%) in a standard state (0°C, 100 kPa).

A volume ratio (gas-liquid volume ratio) between the culture solution 11 and the gas 12 is not particularly limited. A volume of the culture solution 11 may be larger than a volume of the gas 12. The volume of the culture solution 11 may be smaller than the volume of the gas 12. The culture solution 11 and the gas 12 may have the same volume as each other. The gas-liquid volume ratio may be, for example, in a range of 1:9 to 9:1.

A shape of the culture tank 1 is not particularly limited, and may be, for example, a rectangular parallelepiped shape, a cylindrical shape, a rectangular cylindrical shape, or the like. A material of the culture tank 1 is not particularly limited, and may be a resin, rubber, glass, a metal, or the like.

The drive unit 2 causes the culture tank 1 to perform a shaking operation and thereby causes the culture solution 11 to flow while being into contact with the gas 12. Therefore, the drive unit 2 can agitate the culture solution 11 without coming into contact with the culture solution 11. Since the drive unit 2 is used, contamination of the culture solution 11 due to an agitating member can be avoided compared to a case in which an agitating member such as an agitating blade, an agitating rod, and an agitating bar is used. Another advantage of the drive unit 2 is that time and effort such as for replacing the agitating member are not necessary. The drive unit 2 includes, for example, a drive unit main body such as a motor, and a conversion mechanism that converts motion of the drive unit main body into reciprocating motion, rotational motion, or the like. The drive unit main body and the conversion mechanism are an example of a tank shaking-type drive device. The tank shaking-type drive device shakes the culture tank 1.

The shaking operation that the drive unit 2 applies to the culture tank 1 may be a reciprocating motion, a rotational motion, or the like. A direction of the reciprocating motion may be, for example, a horizontal direction (left-right direction), a vertical direction (up-down direction), or an inclined direction therebetween. The rotational motion may be a rotation motion in which a rotating shaft passes through a center of gravity of the culture solution 11. The rotational motion may be a revolving motion in which the rotating shaft passes through a position away from the center of gravity of the culture solution 11. The drive unit 2 may cause two or more types of motions having different types or directions to act on the culture tank 1 at the same time. The drive unit 2 may change the type or direction of the motion along with the passage of time.

The controller 3 controls an operation of the drive unit 2. The controller 3 can control, for example, the number of shaking operations that the drive unit 2 applies to the culture tank 1 per unit time. The controller 3 can also control a magnitude, a type, a direction, or the like of the shaking operation. The controller 3 transmits a control signal to the drive unit 2 via a transmission line 31 to operate the drive unit 2. The "number of shaking operations per unit time" is a shaking speed.

The controller 3 may include a processing device such as a controller to control devices based on received information. The controller 3 may include a storage device, an input device, an output device, a processing program, or the like. An automatic control is possible by using the controller 3. A transmission/reception method of the control signal is not particularly limited, but may be wireless or wired.

### [Cell culture method] (First embodiment)

A method of culturing cells using the cell culture device 10 will be described. Cells contained in the culture solution 11 may be animal cells, plant cells, insect cells, bacteria, yeast, or the like. Examples of animal cells include Chinese hamster ovary (CHO) cells for biopharmaceutical production, HeLa cells, COS cells, iPS cells for regenerative medicine applications, stem cells such as mesenchymal stem cells, differentiated tissue cells, or the like. Cells can produce various substances useful as, for example, pharmaceuticals, foods, cosmetics, their raw materials, or the like.

In a case in which cells with adhesion dependence (anchorage dependence) are cultured, it may be necessary to secure an anchorage as a substitute for other cells or extracellular matrix. In that case, anchorage materials (microcarriers) capable of floating in the solution may be contained in the culture solution 11. Cells can adhere to the microcarriers. The microcarriers may be porous, capsules, hollow fibers, or the like.

The microcarrier may be a fibrous scaffold such as nanofibers or a particulate scaffold such as microbeads. Examples of materials of the microcarriers include polysaccharides such as cellulose, hemicellulose, chitin, chitosan, dextran, agarose, and alginate, proteins such as collagen and gelatin, synthetic polymers such as polystyrene, polyacrylamide, polycarbonate, polyethylene, polypropylene, and silicone, inorganic materials such as glass, silica, and alumina, or the like. A surface of the microcarrier may be coated with a coating material according to types of cells, culture conditions, or the like. Examples of coating materials include proteins such as collagen, gelatin, and keratin.

When the controller 3 applies a shaking operation to the culture tank 1 using the drive unit 2, the culture solution 11 is shaken and flows while coming into contact with the gas 12 at the liquid surface L. For example, the culture solution 11 is in an inclined state such as liquid surfaces LA and LB with respect to the horizontal liquid surface L in a stationary state. The liquid surface L continuously changes, for example, to repeat the liquid surfaces L, LA, L, and LB along with the passage of time. A culture method performed by causing the culture tank 1 to perform the shaking operation is called "shaking culture".

As the culture solution 11 flows, dissolution of the gas 12 (oxygen-containing gas such as air) into the culture solution 11 is promoted. Therefore, oxygen is supplied to the culture solution 11. At least some of the supplied oxygen is consumed by the cells.

The controller 3 controls the operation of the drive unit 2 so that the shaking speed of the culture tank 1 increases continuously or intermittently along with the passage of time.

FIG. 2 is a diagram showing a first example of temporal change in shaking speed. In the example shown in FIG. 2, the shaking speed increases continuously. Specifically, the shaking speed increases according to a linear function (linearly). When a first period P1 and a second period P2 which are different in time series are compared, the shaking speed in the second period P2 is higher (faster) than the shaking speed in the first period P1. The second period P2 is a period after the first period P1 in time series.

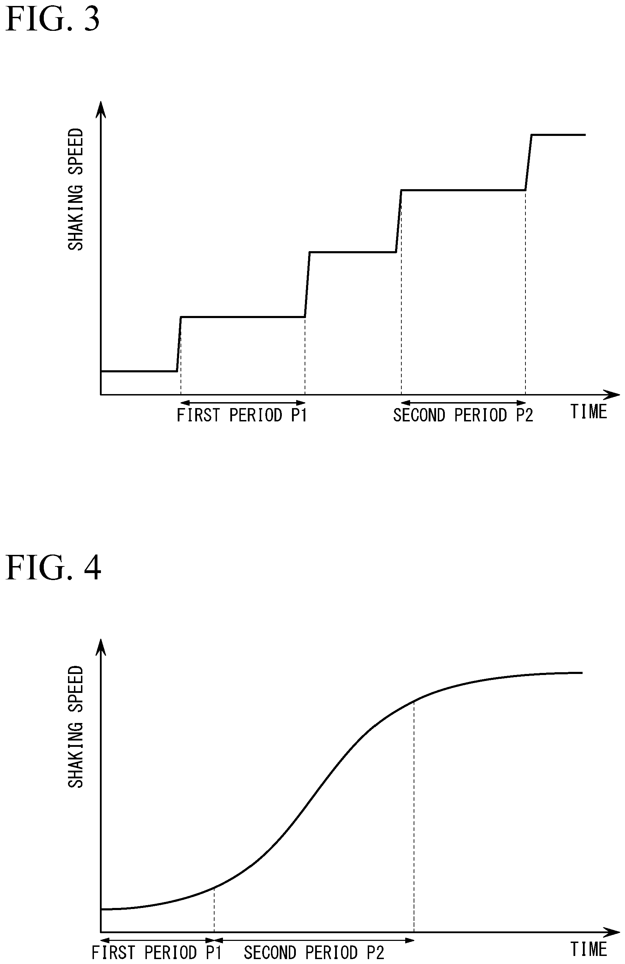

FIG. 3 is a diagram showing a second example of temporal change in shaking speed. In the example shown in FIG. 3, the shaking speed increases intermittently (discontinuously). Specifically, regarding the shaking speed, a period in which it increases and a period in which it remains constant (periods in which the shaking speed does not increase) are repeated. In this example, periods in which shaking speed increases occur at intervals. Both the first period P1 and the second period P2 are periods in which the shaking speed is constant. Also in this example, the shaking speed in the second period P2 is higher (faster) than the shaking speed in the first period P1.

FIG. 4 is a diagram showing a third example of temporal change in shaking speed. In the example shown in FIG. 4, the shaking speed increases continuously. A first period P1 corresponds to a so-called induction phase. A second period P2 corresponds to a so-called logarithmic growth phase. In the second period P2, the shaking speed increases exponentially. Also in this example, the shaking speed in the second period P2 is higher (faster) than the shaking speed in the first period P1. In this example, it is assumed that a cell growth process includes the induction phase, the logarithmic growth phase, a stationary phase, and a death phase.

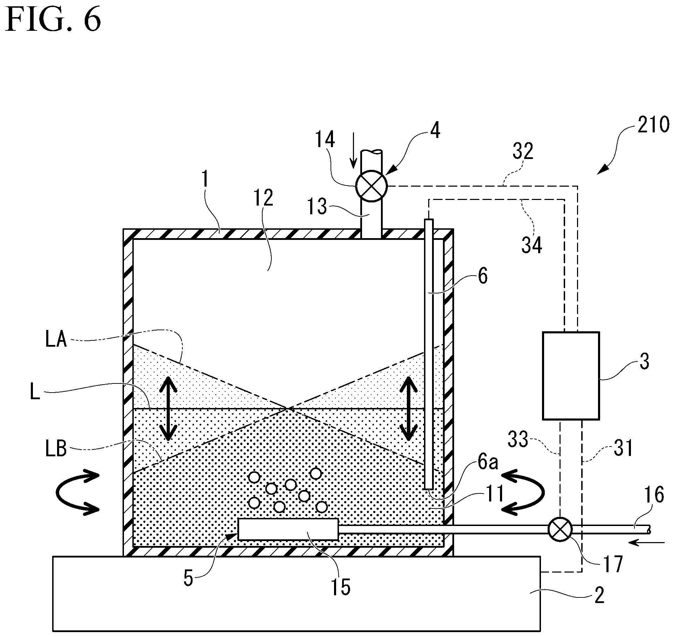

Regarding a rate of increase (shaking acceleration) in the number of shaking operations, it is desirable to determine the rate of increase in the number of shaking operations at which oxygen can be supplied to the cells in just the right amount by conducting a preliminary test. Regarding a maximum shaking speed of the culture tank 1 that is allowable under cell culture conditions, it is desirable to determine the maximum shaking speed of the culture tank 1 at which oxygen can be supplied to the cells in just the right amount by conducting a preliminary test. Regarding the shaking acceleration, the shaking acceleration can also be determined in accordance with a change in dissolved oxygen concentration in the culture solution 11. The dissolved oxygen concentration in the culture solution 11 can be measured using a dissolved oxygen meter (see FIG. 6).

### [Effects of cell culture device and cell culture method of first embodiment]

The cell culture device 10 includes the controller 3 that controls the operation of the drive unit 2 so that the shaking speed of the culture tank 1 increases continuously or intermittently.

Since the controller 3 slows down the shaking speed of the culture tank 1 in an early stage of the culture process in which the number of cells in the culture solution 11 is small, the cells being affected by excessive oxygen can be suppressed. For example, the cells being susceptible to oxygen damage can be avoided. Since the cells in the culture solution 11 proliferate along with the passage of time, an amount of oxygen consumed by the cells increases, but when the shaking speed of the culture tank 1 is controlled by the controller 3, the dissolved oxygen concentration in the culture solution 11 becomes appropriate, and sufficient oxygen is provided to the cells. In this way, according to the cell culture device 10, oxygen can be supplied in just the right amount in accordance with the number of cells in the culture solution 11. Therefore, a physiological state of the cells can be satisfactorily maintained.

### [Cell culture device] (Second embodiment)

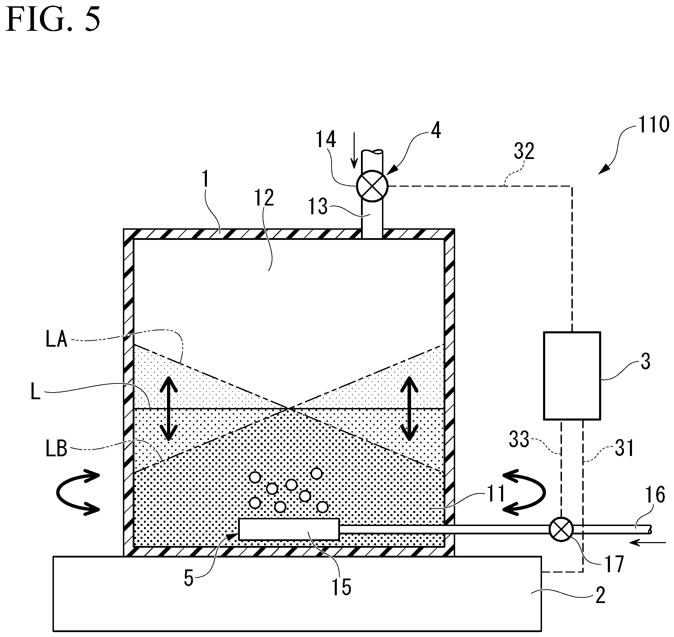

FIG. 5 is a schematic view showing a cell culture device 110 of a second embodiment. Furthermore, components common to other embodiments are denoted by the same reference signs and description thereof will be omitted.

As shown in FIG. 5, the cell culture device 110 includes a culture tank 1, a drive unit 2, a controller 3, a first supply system 4, and a second supply system 5. The cell culture device 110 differs from the cell culture device 10 of the first embodiment (see FIG. 1) in that the first supply system 4 and the second supply system 5 are provided.

The controller 3 controls operations of the drive unit 2, the first supply system 4, and the second supply system 5.

The first supply system 4 includes a supply pipe line 13 (supply route) and a flow rate adjuster 14.

The supply pipe line 13 is connected to an upper portion of the culture tank 1. The supply pipe line 13 supplies an oxygen-containing gas supplied from a supply source (not shown in the drawings) to a gas-phase space of the culture tank 1. An oxygen concentration of the oxygen-containing gas can be higher than 0% and 100% or lower. The oxygen concentration described above may be higher or lower than an oxygen concentration of air (approximately 21%).

A discharge hole (not shown in the drawings) for discharging the gas inside the culture tank 1 is formed in the culture tank 1.

The flow rate adjuster 14 adjusts an amount of the oxygen-containing gas supplied through the supply pipe line 13. The flow rate adjuster 14 is, for example, a valve, a pump, or the like. In a case in which the flow rate adjuster 14 is a valve, the flow rate adjuster 14 adjusts an amount of the oxygen-containing gas supplied through the supply pipe line 13 by adjusting a flow path opening area. The controller 3 transmits a control signal to the flow rate adjuster 14 via a transmission line 32 to operate the flow rate adjuster 14.

The second supply system 5 includes a gas disperser 15, a supply pipe line 16 (supply route), and a flow rate adjuster 17.

The gas disperser 15 is provided in a culture solution 11 in the culture tank 1. The gas disperser 15 is, for example, a tubular body formed of a porous material. The gas disperser 15 can supply the oxygen-containing gas into the culture solution 11 by dispersing (bubbling). The oxygen-containing gas is supplied to the culture solution 11 as bubbles. It is desirable that the second supply system 5 have a higher capacity to supply oxygen to the culture solution 11 (capacity to supply oxygen dissolved in the culture solution 11) than that of the first supply system 4.

The supply pipe line 16 supplies an oxygen-containing gas supplied from the supply source (not shown in the drawings) to the gas disperser 15. The oxygen-containing gas is, for example, air. The oxygen concentration of the oxygen-containing gas may be higher or lower than an oxygen concentration of air. A concentration of oxygen contained in the oxygen-containing gas may be higher than 0% and 100% or lower. The oxygen concentration of the oxygen-containing gas may be, for example, approximately 21% to 100%.

The flow rate adjuster 17 adjusts an amount of the oxygen-containing gas supplied through the supply pipe line 16. The flow rate adjuster 17 is, for example, a valve, a pump, or the like. In a case in which the flow rate adjuster 17 is a valve, the flow rate adjuster 17 adjusts an amount of the oxygen-containing gas supplied through the supply pipe line 16 by adjusting a flow path opening area. The controller 3 transmits a control signal to the flow rate adjuster 17 via a transmission line 33 to operate the flow rate adjuster 17.

The controller 3 can also control an operation of at least one of the first supply system 4 and the second supply system 5 based on a detected value of the dissolved oxygen concentration in the culture solution 11.

In a case in which at least one of the first supply system 4 and the second supply system 5 is controlled by, for example, a proportional integral differential control (PID) control, when the dissolved oxygen concentration in the culture solution 11 is lower than a set value, an oxygen-containing gas with a high oxygen concentration can be supplied to increase the dissolved oxygen concentration, and when the dissolved oxygen concentration in the culture solution 11 is higher than the set value, an oxygen-containing gas with a low oxygen concentration can be supplied to lower the dissolved oxygen concentration. The dissolved oxygen concentration in the culture solution 11 can be measured using a dissolved oxygen meter (see FIG. 6).

### [Cell culture method] (Second embodiment)

A method of culturing cells using the cell culture device 110 will be described.

### (First step: oxygen supply by tank shaking operation)

The controller 3 applies a shaking operation to the culture tank 1 using the drive unit 2 to shake the culture solution 11. The controller 3 controls an operation of the drive unit 2 so that, for example, a shaking speed (the number of shaking operations per unit time) of the culture tank 1 increases along with the passage of time (see FIGS. 2 to 4). The shaking speed of the culture tank 1 may be increased continuously or may be increased intermittently. As the culture solution 11 flows, dissolution of the gas 12 (oxygen-containing gas such as air) into the culture solution 11 is promoted. At least some of the supplied oxygen is consumed by the cells.

### (Second step: oxygen supply by first supply system)

The controller 3 drives the first supply system 4 when it is detected that the shaking speed (the number of shaking operations per unit time) of the culture tank 1 due to the drive unit 2 has reached a specific value. Specifically, when the controller 3 detects that the shaking speed of the culture tank 1 due to the drive unit 2 has reached the specific value or that the dissolved oxygen concentration in the culture solution 11 has reached the set value, the controller 3 operates the flow rate adjuster 14 to supply the oxygen-containing gas with a high oxygen concentration into the culture tank 1 through the supply pipe line 13.

Since the oxygen-containing gas with a high oxygen concentration is supplied into the culture tank 1, an oxygen concentration of the gas 12 in the culture tank 1 increases. Therefore, an amount of oxygen supplied to the culture solution 11 increases. At least some of the supplied oxygen is consumed by the cells.

In the second step, the shaking speed of the culture tank 1 due to the drive unit 2 is maintained at the maximum value or the set value.

The controller 3 controls an operation of the flow rate adjuster 14 so that, for example, a flow rate of the oxygen-containing gas increases along with the passage of time. The increase in the flow rate of the oxygen-containing gas according to the passage of time may be continuous or intermittent. For example, when the first period and the second period after the first period, which are different in time series, are compared, a flow rate of the oxygen-containing gas in the second period is higher than a flow rate of the oxygen-containing gas in the first period. In order to operate the flow rate adjuster 14 so that the flow rate of the oxygen-containing gas increases along with the passage of time, for example, the flow path opening area in the flow rate adjuster 14 need only be increased along with the passage of time.

Regarding a rate of increase in the flow rate of the oxygen-containing gas, it is desirable to determine the rate of increase at which oxygen can be supplied to the cells in just the right amount by conducting a preliminary test. The rate of increase in the flow rate of the oxygen-containing gas can also be determined in accordance with a change in dissolved oxygen concentration in the culture solution 11.

The phrase "the shaking speed due to the drive unit 2 reaches a specific value" refers to, for example, (i) a rotation speed of the drive unit main body (motor) of the drive unit 2 reaches an upper limit of an operational capacity of the drive unit main body, (ii) the tank shaking speed reaches the set value, (iii) the dissolved oxygen concentration in the culture solution 11 reaches the set value, or the like. At this time, an amount of oxygen supplied due to the tank shaking operation of the culture tank 1 is a specific value (for example, the maximum value).

### (Third step: oxygen supply by second supply system)

The controller 3 drives the second supply system 5 in accordance with the fact that an amount of oxygen supplied by the first supply system 4 has reached the maximum or set value. Specifically, the controller 3 drives the second supply system 5 when it is detected that (i) the amount of the oxygen-containing gas supplied by the first supply system 4 has reached a maximum supply capacity, (ii) the supply amount of the oxygen-containing gas has reached the set value, (iii) the dissolved oxygen concentration in the culture solution 11 has reached the set value, or the like. Specifically, when the controller 3 detects at least one of (i) to (iii), the controller 3 operates the flow rate adjuster 17 to supply the oxygen-containing gas (for example, air) to the gas disperser 15 through the supply pipe line 16. The gas disperser 15 supplies the oxygen-containing gas into the culture solution 11 by dispersing (bubbling).

Since the oxygen-containing gas is supplied into the culture solution 11, the amount of oxygen supplied to the culture solution 11 increases.

The controller 3 controls an operation of the flow rate adjuster 17 so that, for example, the flow rate of the oxygen-containing gas increases continuously or intermittently along with the passage of time. For example, when the first period and the second period after the first period, which are different in time series, are compared, a flow rate of the oxygen-containing gas in the second period is preferably higher than a flow rate of the oxygen-containing gas in the first period.

In order to operate the flow rate adjuster 17 so that the flow rate of the oxygen-containing gas increases along with the passage of time, for example, the flow path opening area in the flow rate adjuster 17 need only be increased along with the passage of time.

Regarding the rate of increase in the flow rate of the oxygen-containing gas, it is desirable to determine the rate of increase at which oxygen can be supplied to the cells in just the right amount by conducting a preliminary test. The rate of increase in the flow rate of the oxygen-containing gas can also be determined in accordance with a change in dissolved oxygen concentration in the culture solution 11.

In the third step, the number of shaking operations of the culture tank 1 per unit time due to the drive unit 2 is maintained at the maximum value or the set value. The amount of the oxygen-containing gas supplied by the first supply system 4 is also maintained at the maximum value or the set value.

The phrase "the amount of the oxygen-containing gas supplied by the first supply system 4 reaches the maximum supply capacity" means that, for example, a pump for sending the oxygen-containing gas through the supply pipe line 16 reaches an upper limit of an operational capacity thereof or reaches an upper limit of the supply amount of the oxygen-containing gas based on culture conditions or a structure of the culture tank 1. At this time, the amount of oxygen supplied by the first supply system 4 becomes maximum.

### [Effects of cell culture device and cell culture method of second embodiment]

In addition to the same effects similar to the first embodiment, the cell culture device 110 has the following effects.

The controller 3 supplies a high-concentration oxygen-containing gas using the first supply system 4 after the tank shaking speed based on the drive unit 2 has reached a specific value. After the amount of oxygen supply of the first supply system 4 reaches the maximum or a specific value, the second supply system 5 is used to further supply the oxygen-containing gas. Although an amount of oxygen consumed by the cells in the culture solution 11 increases, since oxygen can be supplied by the first supply system 4 and the second supply system 5, the dissolved oxygen concentration in the culture solution 11 becomes appropriate, and sufficient oxygen is provided to the cells. Therefore, a physiological state of the cells can be satisfactorily maintained.

### [Cell culture device] (Third embodiment)

FIG. 6 is a schematic view showing a cell culture device 210 of a third embodiment. Furthermore, components common to other embodiments are denoted by the same reference signs and description thereof will be omitted.

As shown in FIG. 6, the cell culture device 210 includes a culture tank 1, a drive unit 2, a controller 3, a first supply system 4, a second supply system 5, and a dissolved oxygen meter 6. The cell culture device 210 differs from the cell culture device 110 (see FIG. 5) of the second embodiment in that the dissolved oxygen meter 6 is provided. The dissolved oxygen meter 6 is, for example, a diaphragm-type dissolved oxygen meter.

The dissolved oxygen meter 6 is provided such that a dissolved oxygen concentration sensor 6a is disposed at a lower portion in the culture tank 1. Therefore, the dissolved oxygen concentration sensor 6a is immersed in a culture solution 11. The dissolved oxygen concentration sensor 6a detects a dissolved oxygen concentration of the culture solution 11 and transmits a detection signal to the controller 3 via a transmission line 34.

### [Cell culture method] (Third embodiment)

A method of culturing cells using the cell culture device 210 will be described.

### (First step: oxygen supply by tank shaking operation)

The controller 3 applies a shaking operation to the culture tank 1 using the drive unit 2 to shake the culture solution 11. The controller 3 controls an operation of the drive unit 2 so that, for example, a shaking speed of the culture tank 1 increases continuously or intermittently along with the passage of time (see FIGS. 2 to 4).

The controller 3 can also control the shaking speed of the culture tank 1 based on a detected value of the dissolved oxygen concentration by the dissolved oxygen meter 6. For example, when the detected value is lower than a set value, the shaking speed of the culture tank 1 can be increased, and when the detected value is higher than the set value, the shaking speed of the culture tank 1 can be lowered.

The controller 3 controls driving and stopping of the first supply system 4 based on the detected value of the dissolved oxygen concentration by the dissolved oxygen meter 6. For example, if the detected value of the dissolved oxygen concentration is equal to or higher than a preset set value, the controller 3 does not drive the first supply system 4.

### (Second step: oxygen supply by first supply system)

The controller 3 drives the first supply system 4 in a case in which the detected value of the dissolved oxygen concentration has fallen below the preset set value. Specifically, the controller 3 operates the flow rate adjuster 14 to supply the oxygen-containing gas with a high oxygen concentration into the culture tank 1 through the supply pipe line 13. The controller 3 controls an operation of the flow rate adjuster 14 so that, for example, a flow rate of the oxygen-containing gas increases continuously or intermittently along with the passage of time.

The controller 3 controls driving and stopping of the second supply system 5 based on the detected value of the dissolved oxygen concentration by the dissolved oxygen meter 6. For example, if the detected value of the dissolved oxygen concentration is equal to or higher than the preset set value, the controller 3 does not drive the second supply system 5.

### (Third step: oxygen supply by second supply system)

In a case in which the detected value of the dissolved oxygen concentration has fallen below the preset set value, the controller 3 drives the second supply system 5. Specifically, the controller 3 operates the flow rate adjuster 17 to supply the oxygen-containing gas (for example, air) to the gas disperser 15 through the supply pipe line 16. The controller 3 controls an operation of the flow rate adjuster 17 so that, for example, the flow rate of the oxygen-containing gas increases continuously or intermittently along with the passage of time.

### [Effects of cell culture device and cell culture method of third embodiment]

In addition to the same effects similar to the first embodiment, the cell culture device 210 has the following effects.

The controller 3 supplies the high-concentration oxygen-containing gas using the first supply system 4 and the second supply system 5 based on the detected value of the dissolved oxygen concentration by the dissolved oxygen meter 6. Although an amount of oxygen consumed by the cells in the culture solution 11 increases, since oxygen can be supplied by the first supply system 4 and the second supply system 5, the dissolved oxygen concentration in the culture solution 11 becomes appropriate.

Since the controller 3 controls driving and stopping of the first supply system 4 and the second supply system 5 based on the detected value of the dissolved oxygen concentration by the dissolved oxygen meter 6, the dissolved oxygen concentration of the culture solution 11 becomes appropriate, and oxygen is provided to the cells in just the right amount. Therefore, a physiological state of the cells can be satisfactorily maintained.

### [Cell culture device] (Fourth embodiment)

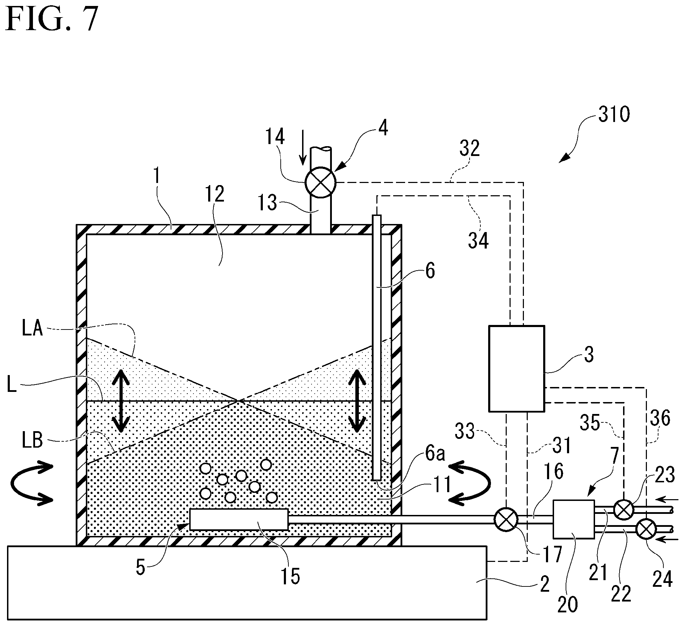

FIG. 7 is a schematic view showing a cell culture device 310 of a fourth embodiment. Furthermore, components common to other embodiments are denoted by the same reference signs and description thereof will be omitted.

As shown in FIG. 7, the cell culture device 310 includes a culture tank 1, a drive unit 2, a controller 3, a first supply system 4, a second supply system 5, a dissolved oxygen meter 6, and a third supply system 7. The cell culture device 310 differs from the cell culture device 210 (see FIG. 6) of the third embodiment in that it includes the third supply system 7.

The controller 3 controls operations of the drive unit 2, the first supply system 4, the second supply system 5, and the third supply system 7.

The controller 3 can control operations of the drive unit 2, the first supply system 4, the second supply system 5, and the third supply system 7 by PID control based on, for example, a detected value of a dissolved oxygen concentration by the dissolved oxygen meter 6.

For example, the controller 3 can increase a drive speed of the drive unit 2 when the detected value of the dissolved oxygen concentration in a culture solution 11 is lower than a set value, and reduce the drive speed of the drive unit 2 when the detected value of the dissolved oxygen concentration in the culture solution 11 is higher than the set value.

For example, the controller 3 can increase an amount of the oxygen-containing gas supplied from each of the supply systems when the detected value of the dissolved oxygen concentration in the culture solution 11 is lower than the set value, and reduce an amount of the oxygen-containing gas supplied from each of the supply systems when the detected value of the dissolved oxygen concentration in the culture solution 11 is higher than the set value.

For example, the controller 3 can increase the dissolved oxygen concentration by supplying the oxygen-containing gas with a high oxygen concentration when the detected value of the dissolved oxygen concentration in the culture solution 11 is lower than the set value, and lower the dissolved oxygen concentration by supplying the oxygen-containing gas with a low oxygen concentration when the detected value of the dissolved oxygen concentration in the culture solution 11 is higher than the set value.

The third supply system 7 includes an adjustment unit 20, a first supply pipe line 21, a second supply pipe line 22, a first flow rate adjuster 23, and a second flow rate adjuster 24. The third supply system 7 adjusts an oxygen concentration of the oxygen-containing gas supplied to a gas disperser 15 through a supply pipe line 16.

The first supply pipe line 21 supplies a first gas supplied from a supply source (not shown in the drawings) to the adjustment unit 20. The first gas is, for example, air. The first gas may be an oxygen-containing gas or a gas without containing oxygen.

The first flow rate adjuster 23 adjusts an amount of the first gas supplied through the first supply pipe line 21. The first flow rate adjuster 23 is, for example, a valve, a pump, or the like. In a case in which the first flow rate adjuster 23 is a valve, the first flow rate adjuster 23 adjusts the amount of the first gas supplied through the first supply pipe line 21 by adjusting a flow path opening area. The controller 3 transmits a control signal to the first flow rate adjuster 23 via a transmission line 35 to operate the first flow rate adjuster 23.

The second supply pipe line 22 supplies a second gas supplied from the supply source (not shown in the drawings) to the adjustment unit 20. The second gas is an oxygen-containing gas. An oxygen concentration of the second gas may be higher or lower than the oxygen concentration of the first gas. The oxygen concentration of the second gas, for example, may be higher than 0% and 100% or lower, and may be approximately 21% to 100%.

The second flow rate adjuster 24 adjusts an amount of the second gas supplied through the second supply pipe line 22. The second flow rate adjuster 24 is, for example, a valve, a pump, or the like. In a case in which the second flow rate adjuster 24 is a valve, the second flow rate adjuster 24 adjusts the amount of the second gas supplied through the second supply pipe line 22 by adjusting a flow path opening area. The controller 3 transmits a control signal to the second flow rate adjuster 24 via a transmission line 36 to operate the second flow rate adjuster 24.

A base end of the supply pipe line 16, a distal end of the first supply pipe line 21, and a distal end of the second supply pipe line 22 are connected to the adjustment unit 20. The adjustment unit 20 can send a mixed gas of the first gas from the first supply pipe line 21 and the second gas from the second supply pipe line 22 to the gas disperser 15 through the supply pipe line 16.

The controller 3 can adjust an oxygen concentration of the mixed gas by operating the first flow rate adjuster 23 and the second flow rate adjuster 24, and adjusting a mixing ratio of the first gas and the second gas.

### [Cell culture method] (Fourth embodiment)

A method of culturing cells using the cell culture device 310 will be described.

### (First step: oxygen supply by tank shaking operation)

The controller 3 applies a shaking operation to the culture tank 1 using the drive unit 2 to shake the culture solution 11. The controller 3 controls an operation of the drive unit 2 so that, for example, a shaking speed of the culture tank 1 increases continuously or intermittently along with the passage of time (see FIGS. 2 to 4). At least some of the supplied oxygen is consumed by the cells.

### (Second step: oxygen supply by first supply system)

When it is detected that the shaking speed of the culture tank 1 due to the drive unit 2 has reached the maximum or set value, the controller 3 drives the first supply system 4. Specifically, when it is detected that the shaking speed of the culture tank 1 due to the drive unit 2 has reached the maximum or set value, the controller 3 operates a flow rate adjuster 14 to supply the oxygen-containing gas with a high oxygen concentration into the culture tank 1 through a supply pipe line 13. The controller 3 controls an operation of the flow rate adjuster 14 so that, for example, a flow rate of the oxygen-containing gas increases continuously or intermittently along with the passage of time. At least some of the supplied oxygen is consumed by the cells.

In the second step, the number of shaking operations per unit time due to the drive unit 2 is maintained at the maximum value or the set value.

### (Third step: oxygen supply by second supply system)

When it is detected that the amount of the oxygen-containing gas supplied by the first supply system 4 has reached a maximum supply capacity or that the dissolved oxygen concentration in the culture solution 11 has reached the set value, the controller 3 drives the second supply system 5. Specifically, when the controller 3 detects that the amount of the oxygen-containing gas supplied by the first supply system 4 has reached the maximum supply capacity or that the dissolved oxygen concentration in the culture solution 11 has reached the set value, the controller 3 operates the flow rate adjuster 17 to supply the oxygen-containing gas (for example, air) to the gas disperser 15 through the supply pipe line 16. The gas disperser 15 supplies the oxygen-containing gas into the culture solution 11 by dispersing (bubbling). The controller 3 controls an operation of the flow rate adjuster 17 so that, for example, the flow rate of the oxygen-containing gas increases continuously or intermittently along with the passage of time. At least some of the supplied oxygen is consumed by the cells.

Similar to the cell culture method of the third embodiment, the controller 3 may control driving and stopping of the second supply system 5 based on the detected value of the dissolved oxygen concentration by the dissolved oxygen meter 6. For example, if the detected value of the dissolved oxygen concentration is equal to or higher than the preset set value, the controller 3 does not drive the second supply system 5. In a case in which the detected value of the dissolved oxygen concentration is lower than the preset set value, the second supply system 5 is driven.

In the third step, the number of shaking operations per unit time due to the drive unit 2 is maintained at the maximum value or the set value. The amount of the oxygen-containing gas supplied by the first supply system 4 is also maintained at the maximum value or the set value.

### (Fourth step: oxygen supply by third supply system)

When it is detected that an amount of the oxygen-containing gas supplied by the second supply system 5 has reached a specific value (for example, a maximum value) of the supply capacity or has reached a set supply amount, or that the dissolved oxygen concentration in the culture solution 11 has reached the set value, the controller 3 drives the third supply system 7. Specifically, when the controller 3 detects that the amount of the oxygen-containing gas supplied by the second supply system 5 has reached the specific value of the supply capacity or has reached the set supply amount, or that the dissolved oxygen concentration in the culture solution 11 has reached the set value, the controller 3 operates the first flow rate adjuster 23 and the second flow rate adjuster 24 to adjust an oxygen concentration of the mixed gas. Specifically, a proportion of the second gas in the mixed gas is increased by adjusting the flow path opening area in the first flow rate adjuster 23 and the second flow rate adjuster 24. For example, the flow path opening area in the second flow rate adjuster 24 is made larger while the flow path opening area in the first flow rate adjuster 23 is made smaller. Therefore, since the proportion of the second gas in the mixed gas increases, an oxygen concentration of the oxygen-containing gas sent to the gas disperser 15 increases.

Regarding a rate of rise in oxygen concentration, it is desirable to determine the rate of rise at which oxygen can be supplied to the cells in just the right amount by conducting a preliminary test.

The phrase "the amount of the oxygen-containing gas supplied by the second supply system 5 reaches the specific value of the supply capacity" means that, for example, a pump for sending the oxygen-containing gas through the supply pipe line 16 reaches an upper limit of an operational capacity thereof. At this time, an amount of oxygen supplied by the second supply system 5 becomes a specific value (for example, a maximum value).

For example, the controller 3 can control operations of the first flow rate adjuster 23 and the second flow rate adjuster 24 so that the oxygen concentration of the oxygen-containing gas increases along with the passage of time.

In order to operate the first flow rate adjuster 23 and the second flow rate adjuster 24 so that the oxygen concentration of the oxygen-containing gas increases along with the passage of time, for example, a ratio of the flow path opening area of the second flow rate adjuster 24 to the flow path opening area of the first flow rate adjuster 23 need only be increased along with the passage of time.

In the fourth step, the shaking speed of the culture tank 1 due to the drive unit 2 is maintained at the maximum value or the set value. The amount of the oxygen-containing gas supplied by the first supply system 4 is also maintained at the maximum value or the set value. The amount of the oxygen-containing gas supplied by the second supply system 5 is also maintained at the maximum value or the set value.

### [Effects of cell culture device and cell culture method of fourth embodiment]

In addition to the same effects similar to the first embodiment and the second embodiment, the cell culture device 310 has the following effects.

After the shaking speed of the culture tank 1 due to the drive unit 2 reaches the maximum or set value, the controller 3 supplies a high-concentration oxygen-containing gas using the first supply system 4. After the amount of oxygen supply of the first supply system 4 reaches the maximum or set value, the second supply system 5 is used to further supply the oxygen-containing gas. After the amount of oxygen supply of the second supply system 5 reaches the maximum or set value, the third supply system 7 is used to increase the oxygen concentration of the oxygen-containing gas.

According to the cell culture device 310, although an amount of oxygen consumed by the cells in the culture solution 11 increases, since oxygen can be supplied by the first supply system 4, the second supply system 5, and the third supply system 7, the dissolved oxygen concentration in the culture solution 11 becomes appropriate, and sufficient oxygen is provided to the cells. Therefore, a physiological state of the cells can be satisfactorily maintained.

The supply pipe line connected to the culture tank 1 may be a flexible tube, a hose, or the like, or may be a hard pipe or the like. Examples of the material for the supply pipe line include a resin, rubber, an elastomer, a metal, or the like.

While the invention has been described above with reference to preferred embodiments, the invention is not limited to the above-described embodiments, and various modifications can be made within a range not departing from the gist of the invention. Modifications include additions, substitutions, omissions, and other changes of constituent elements in each embodiment.

In the second to fourth embodiments, an example in which the number of the supply systems that supply oxygen is two or three has been described, but the number of the supply systems is not particularly limited. The number of the supply systems may be one or in plurality (any number of two or more). For example, in addition to the first to third supply systems, a fourth supply system that supplies oxygen to the culture solution through a route different from that of the first to third supply systems may also be used.

The cell culture device may have a configuration in which the second supply system 5 is omitted from the configuration shown in FIG. 5, that is, a configuration in which the culture tank 1, the drive unit 2, the controller 3, and the first supply system 4 are provided.

Examples of cell culture methods include batch culture, feeding culture, perfusion culture, or the like.

The drive unit 2 (see FIG. 1) can also be regarded as one supply system. Therefore, the cell culture device of the embodiment can be rephrased as a cell culture device including a culture tank that stores a culture solution containing cells, and one or more supply systems that supply oxygen to the culture tank.

### REFERENCE SIGNS LIST

1 Culture tank
2 Drive unit
3 Controller
4 First supply system
5 Second supply system
6 Dissolved oxygen meter
7 Third supply system
10, 110, 210, 310 Cell culture device
11 Culture solution
12 Gas

## Claims

1. A cell culture device comprising:
a culture tank that stores a culture solution containing cells;
a drive unit that causes the culture tank to perform a shaking operation and thereby causes the culture solution to flow while being into contact with a gas in the culture tank; and
a controller that controls an operation of the drive unit such that a shaking speed of the culture tank increases continuously or intermittently along with the passage of time.

2. The cell culture device according to claim 1, further comprising a dissolved oxygen meter that measures a dissolved oxygen concentration of the culture solution, wherein
the controller controls the shaking speed of the culture tank based on a detected value of the dissolved oxygen concentration by the dissolved oxygen meter.

3. The cell culture device according to claim 1, further comprising a plurality of supply systems that supply oxygen to the culture tank, wherein
the plurality of supply systems include a first supply system and a second supply system, and
the controller drives the first supply system in accordance with the fact that the shaking speed of the culture tank has reached a specific value, and drives the second supply system in accordance with the fact that an amount of oxygen supplied by the first supply system has reached a maximum or set value.

4. The cell culture device according to claim 3, wherein
the second supply system has a higher capacity to supply oxygen dissolved in the culture solution than that of the first supply system.

5. The cell culture device according to claim 1, further comprising:
a plurality of supply systems that supply oxygen to the culture tank; and
a dissolved oxygen meter that measures a dissolved oxygen concentration of the culture solution, wherein
the supply systems include a first supply system and a second supply system, and
the controller controls driving and stopping of the first supply system based on a detected value of the dissolved oxygen concentration by the dissolved oxygen meter, and controls driving and stopping of the second supply system based on a detected value of the dissolved oxygen concentration by the dissolved oxygen meter.

6. The cell culture device according to any one of claims 3 to 5, wherein
the first supply system supplies an oxygen-containing gas to a gas-phase space of the culture tank, and
the second supply system includes a gas disperser that supplies an oxygen-containing gas into the culture solution.

7. The cell culture device according to claim 6, further comprising a third supply system that adjusts an oxygen concentration of the oxygen-containing gas supplied to the gas disperser.
